# EUROPEAN PATENT APPLICATION

(11) **EP 1 457 180 A1**
(43) Date of publication of application: **15.09.2004**
(21) Application number: 04425133.8
(22) Date of filing: 02.03.2004
(51) Int. Cl.: A61F 11/00

(54) **Passive hearing aid**

(30) Priority: 12.03.2003 IT PD20030046
(71) Applicant: Poletto, Gianluigi, 36070 Castelgomberto VI (IT)
(72) Inventor: Poletto, Gianluigi, 36070 Castelgomberto VI (IT)

(57) **Abstract**

Acoustic apparatus comprising a support at least partially surrounding the head of a listener having one end close to a listener ear. The peculiarity of this apparatus consists in that it comprises at least one conveyor means of sound waves formed in correspondence to said end collecting, intensifying and directing said waves towards the ear drum of said ear. The apparatus solves the drawbacks found in the known state of the art, as it allows a listener to improve the auditory perception of the sound source towards which he is turned.

## Description

The present invention relates to an acoustic apparatus particularly, but not exclusively, useful for individual listening of music in domestic rooms, concerts, canorous performances, theatres etc., except the use as medical aid.

It is known that the auditory perception of a listener will instinctively improve if he puts his hand shaped like a dome immediately behind his ear. In this way, it is obtained a temporary extension of the auricle intended to collect a greater quantity of sound weaves and to convey them to the auditory canal. The result of this action consists in a temporary improvement of the auditory perception. Based on this principle in past years medical aids known as ear-trumpets were diffused to improve the auditory capacity of persons with hearing difficulties.

From the trade are still known the hearing aids generally called headphones. The headphones are an accessory for individual sound-listening. Such headphones are constituted of a pair of receivers or earpieces adaptable to the ears and of a support connecting them over the head.

The principal drawbacks found in the described state of the art consist in that the action of nearing the hand to the ear has the characteristic of being exclusively temporary and that the headphones have no usefulness in particular situations such as canorous performances, concerts, and so on.

The present invention aims to set out an acoustic apparatus that overcomes the drawbacks found in the state of the art.

Within the scope of this aim, an object of the invention consists in setting up an apparatus allowing the listener to improve the auditory perception of a sound source.

Another object of the invention consists in setting out an apparatus of a simplified construction.

This aim, as well as these and other objects appearing afterwards, are achieved by an acoustic apparatus according to first claim.

The acoustic apparatus according to first claim allows the listener to intensify the auditory perception of a sound source due to the action exercised by the conveyor means on the sound weaves.

Furthermore, the acoustic apparatus according to the second and third claim permits to realize the conveyor means in a simplified way due to its structure shaped as a frontally opened semi-shell.

Lastly, the acoustic apparatus according to the fourth and fifth claim allows to direct the entirety of sound weaves collected in the semi-shell towards the ear drum thanks to its parabolic internal configuration and to isolate the listener from disturbing surrounding sound sources thanks to the outer soundproof coating material.

Further features and advantages will become apparent from the description of a preferred embodiment illustrated as indication, but not as limitation, in the attached three drawings where:
- figure 1 is a frontal view of an acoustic apparatus according to the invention;

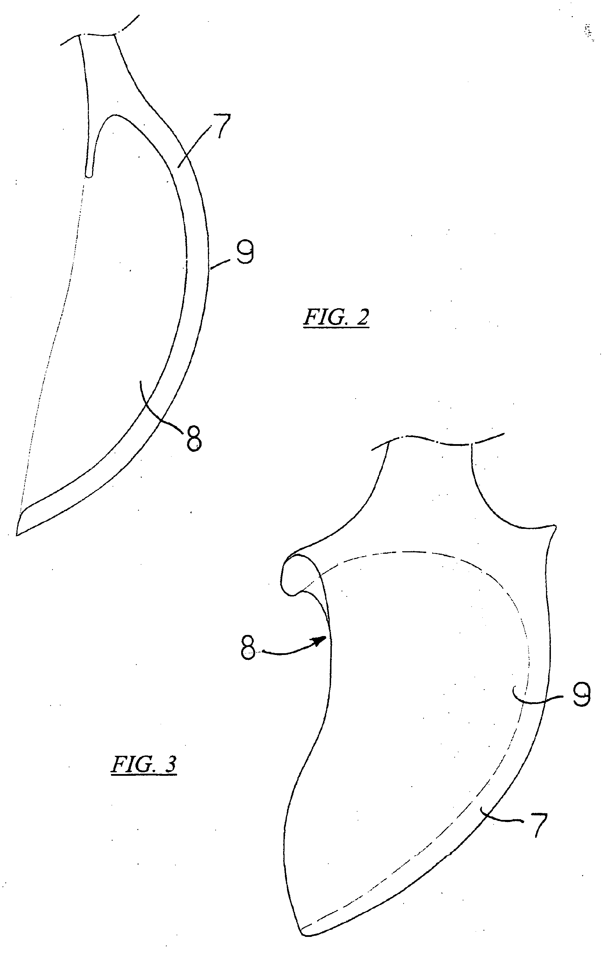

- figure 2 is a detailed front view of the conveyor means of the apparatus of figure 1;
- figure 3 is a detailed side view of the conveyor means of the apparatus of figure 1;
- figure 4 is a frontal view of the apparatus of figure 1 worn by a listener.

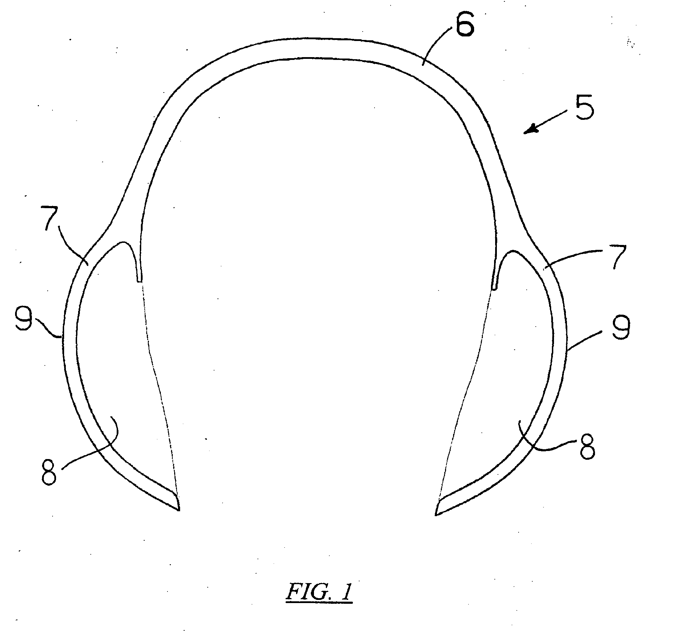

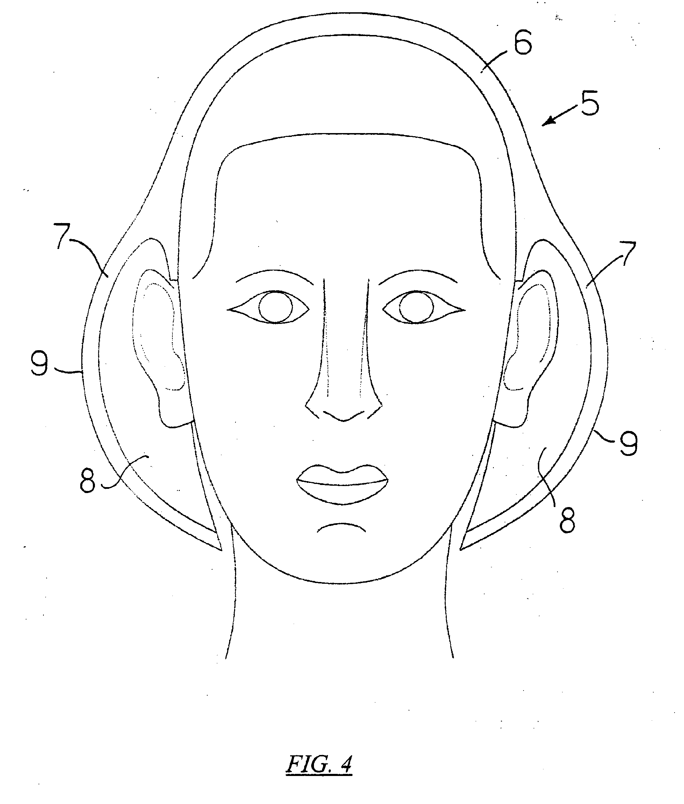

With reference to the figures of the attached drawings, and initially to figure 1, the acoustic apparatus, totally indicated by reference number 5, comprises, according to the known state of the art, a preferably elastic and adjustable support 6. As represented in figure 3, said support 6 partially surrounds the upper part of the listener's head. The ends of support 6 terminate close to the listener's ears. In correspondence to such ends are formed two conveyor means 7 of sound waves. The task of such conveyor means 7 is to collect, intensify and direct the sound waves coming from a source towards the ear drums of the listener's ears. Such conveyor means 7 are constituted of two semi-shells enclosing the auricles of the listener's ears in order to collect the greatest part of the emitted sound waves. Moreover, the front side of said semi-shells are provided with wide openings 8 allowing the sound waves to enter into the semi-shells. Furthermore, the internal configuration of such semi-shells is approximately parabolic in order to concentrate the sound waves collected in the semi-shells towards the ear drums. Lastly, the outside of the semi-shells is covered with a coat 9 of soundproof material in order to prevent that sound waves emitted from disturbing sound sources surrounding the listener influence negatively the auditory perception of the sound waves emitted from the sound source where the listener's attention is directed. This solution shows to be particularly useful in concerts and canorous performances where the acoustic apparatus allows to concentrate the sound waves emitted from the principal source towards the listener's ears and simultaneously to isolate the listener's hearing from the disturbing sound sources such as for example the audience surrounding him.

In practice, it has been observed that the apparatus achieves the intended aim and objects overcoming the drawbacks found in the known state of the art. In fact, it has been observed that the apparatus is able to strengthen the listener's auditory perception by means of an altogether simple structure.

In the practical embodiments, the materials used, the shapes, the dimensions and the execution details may be different from these indicated, but technically equivalent to these.

## Claims

1. Acoustic apparatus comprising a support surrounding at least partially the head of a listener with one end close to one of the two ears of said listener, **characterised in that** it comprises at least one conveyor means of sound waves formed in correspondence to said end which collects, intensifies and directs said waves towards the ear drum of said one of the two ears.

2. Acoustic apparatus, according to claim 1, **characterised in that** said conveyor means comprises a semi-shell which encloses the auricle of said one of the two ears.

3. Acoustic apparatus, according to claim 2, **characterised in that** said semi-shell comprises a frontal opening.

4. Acoustic apparatus, according to claim 2, **characterised in that** said semi-shell comprises an internal configuration being approximately parabolic.

5. Acoustic apparatus, according to claim 2, **characterised in that** said semi-shell comprises an outside coat of soundproof material.
